# EUROPÄISCHE PATENTANMELDUNG

(11) **EP 4 385 490 A1**
(43) Veröffentlichungstag der Anmeldung: **19.06.2024**
(21) Anmeldenummer: 23206675.3
(22) Anmeldetag: 30.10.2023
(51) Int. Cl.: A61K 8/44, A61K 8/67, A61Q 5/02, A61Q 19/10

(54) **FARBSTABILISIERUNG VON KOSMETISCHEN PRODUKTEN**

(30) Priorität: 14.12.2022 DE 102022213655
(71) Anmelder: Henkel AG & Co. KGaA, 40589 Düsseldorf (DE)
(72) Erfinder: Fuhrmann, Guido, 41812 Erkelenz (DE); Schmidt, Istvan, 74081 Heilbronn (DE)

(57) **Zusammenfassung**

Die vorliegende Erfindung betrifft Zusammensetzungen auf der Basis mindestens eines spezifischen Komplexbildners (I) sowie mindestens eines Antioxidans auf Ascorbinsäurebasis, wie beispielsweise Haushaltsprodukte, kosmetische oder pharmazeutische Zusammensetzungen, die eine verbesserte Stabilisierung gegen die schädlichen Auswirkungen von Licht, Hitze und/oder Sauerstoff aufweisen.

## Beschreibung

Die Erfindung betrifft Verbesserungen bei der Stabilisierung insbesondere wasserhaltiger Zusammensetzungen, vorzugsweise von Haushaltsprodukten, kosmetischen und/oder pharmazeutischen Zusammensetzungen. Die Zusammensetzungen umfassen eine Stabilisatormischung aus mindestens einem spezifischen Komplexbildner sowie mindestens einem Antioxidans auf Ascorbinsäurebasis und zeichnen sich durch eine verbesserte Wirksamkeit gegenüber den schädlichen Auswirkungen von Licht, Hitze und/oder Sauerstoff, insbesondere gegenüber Farbveränderungen und/oder Entfärbungen aus.

Handelsübliche Haushaltsprodukte, kosmetische oder pharmazeutische Mittel basieren meist auf Wasser und/oder auf Wasser/Alkoholmischungen und werden zur Steigerung der Produktästhetik oftmals mit für solche Produkte zugelassenen Farbstoffen eingefärbt. Auch die Einarbeitung bestimmter Rohstoffe kann eine unerwünschte Farbveränderung zur Folge haben, weshalb man bestrebt ist, derartigen Produkten eine stabile und ansprechende Färbung zu verleihen.

Die Lagerung von (wasserhaltigen) Erzeugnissen insbesondere bei wechselnder oder starker Lichteinwirkung sowie bei extremen Temperaturschwankungen kann zu einem gewissen Grad der Zersetzung führen, die beispielsweise durch Phasentrennung (beispielsweise bei Emulsionszusammensetzungen), unerwünschte Viskositäts-, Geruchs- und/oder Farbveränderungen erkennbar ist, und die für die Akzeptanz der Erzeugnisse durch den Endverbraucher eine bedeutende Rolle spielt.

Zur Vermeidung oder Minimierung durch Licht bedingter Veränderungen vorgenannter Produkte werden diese oftmals in nicht-transparenten, lichtundurchlässigen Behältnissen abgefüllt und gelagert. Derartige Behältnisse können zudem aus Materialien bestehen, die zur Verbesserung der Lichtundurchlässigkeit UV-Filtersubstanzen, wie beispielsweise Benzotriazol, enthalten. Daneben können auch die Produkte selbst geeignete UV-Filtersubstanzen enthalten.

Nachteilig an UV-Filterzusätzen in Produkten sind zum einen dadurch erhöhte Produktionskosten und zum anderen die Bereitstellung von Produkten, welche zusätzliche synthetische Wirkstoffe enthalten (ohne dass sie dem Verbraucher einen erkennbaren Nutzen bringen, denn für einen Lichtschutz - beispielsweise der Haut - müssten entsprechende Produkte höhere Mengen an UV-Filtersubstanzen enthalten, als sie für die Stabilisierung gegen Farb- oder Geruchsveränderungen erforderlich sind).

In den Offenlegungsschriften DE 197 50 906 und DE 197 39 797 wurde die Verwendung spezieller Triazinderivate zur Stabilisierung organischer Materialien gegen UV-Licht, Sauerstoff und hohe Temperaturen beschrieben.

Aus JP 09078085 A und JP 10237488 A sind kosmetische Reinigungsmittel bekannt, denen zur Verbesserung der Lagerstabilität bei höheren Temperaturen eine Kombination von Komplexbildnern und Antioxidantien hinzugefügt wurde.

DE 10 2006 062 568 betrifft kosmetische oder pharmazeutische Mittel, die als farbstabilisierende Wirkstoffmischung ein phenolisches Antioxidans sowie einen Komplexbildner - vorzugsweise EDTA - enthalten.

Wünschenswert ist neben einer größtmöglichen Flexibilität bei der Auswahl von Verpackungen für vorgenannte Produkte vor allem ein nachhaltigeres Stabilisierungssystem, welches für die Natur weniger kritisch ist als beispielsweise die hochwirksame Wirkstoffkombination aus EDTA und Benzophenone-4. Das Stabilisierungssystem soll eine verbesserte biologische Abbaubarkeit aufweisen und als unbedenklich eingestuft werden (z.B. von Codecheck).

Zudem soll es sich einfach und ohne jedwede negativen Effekte - sei es bei der Herstellung oder der Lagerung - in bestehende Produkte einarbeiten lassen.

Der vorliegenden Erfindung lag daher die Aufgabe zugrunde, gegen UV-Licht-, Sauerstoff- und/oder Temperatureinwirkungen stabilisierte Haushalts-, Kosmetik- und/oder Pharmazieprodukte zur Verfügung zu stellen, die ein Stabilisierungssystem mit verbesserter biologischer Abbaubarkeit - optimalerweise erhältlich aus natürlichen Quellen - umfassen.

Das Stabilisierungssystem soll sich in eine Vielzahl vorgenannter - meist wässriger oder wässrigalkoholischer - Produkte einarbeiten lassen und keine zusätzlichen energetisch aufwändigen Herstellungsschritte und/oder die Einarbeitung zusätzlicher Stabilisatoren erfordern.

Darüber hinaus soll das Stabilisierungssystem kein oder ein nur sehr geringes Irritationspotential aufweisen, um seine Verwendung in einer Vielzahl pharmazeutischer und kosmetischer Zusammensetzungen zu ermöglichen.

Es wurde gefunden, dass die Kombination eines spezifischen Glycinderivats mit Ascorbinsäure und/oder einem Derivat der Ascorbinsäure dafür hervorragend geeignet ist.

Die erfindungsgemäße Wirkstoffkombination ist nachhaltig und aus natürlichen Quellen erhältlich. Sie ist außerdem hervorragend für die Stabilisierung insbesondere wasserhaltiger Haushaltsprodukte, kosmetischer oder pharmazeutischer Produkte gegenüber unerwünschten Farb- oder Geruchsveränderungen geeignet, welche durch UV-Licht- oder Sauerstoffeinwirkung und/oder durch Temperaturschwankungen auftreten können.

Die vorliegende Erfindung sowie bevorzugte Ausführungsformen werden durch die folgenden Aussagen gekennzeichnet:
1. Stabilisierte Zusammensetzung, enthaltend
   a) mindestens einen Komplexbildner der allgemeinen Formel (I) 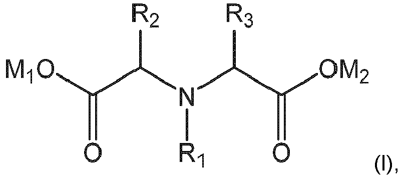 wobei
      - R1 für ein Wasserstoffatom, eine lineare oder verzweigte C₁-C₆-Alkylgruppe, eine lineare oder verzweigte Hydroxy-C₂-C₆-alkylgruppe, eine lineare oder verzweigte Carboxy-C₁-C₆-Alkylgruppe oder für ein physiologisch verträgliches Salz hiervon steht,
      - R2, R3 unabhängig voneinander für ein Wasserstoffatom, eine lineare oder verzweigte C₁-C₆-Alkylgruppe, eine lineare oder verzweigte Hydroxy-C₂-C₆-alkylgruppe, eine lineare oder verzweigte Carboxy-C₁-C₆-Alkylgruppe oder für ein physiologisch verträgliches Salz hiervon stehen,
      - M1, M2 unabhängig voneinander für ein Wasserstoffatom oder ein Äquivalent eines Alkali-, Erdalkali- oder Metallions, bevorzugt für Natrium, Kalium, ½ Magnesium, ½ Calcium, ½ Zink, oder für ein Ammoniumion (NH₄⁺) stehen, und
   b) mindestens ein Antioxidans, ausgewählt aus der Gruppe der Ascorbinsäure, Ascorbinsäuresalze, Ascorbylester oder Mischungen davon.
2. Stabilisierte Zusammensetzung nach Aussage 1, enthaltend Wasser in einem Gewichtsanteil von 5-90 Gew.-% am Gesamtgewicht der Zusammensetzung.
3. Stabilisierte Zusammensetzung nach einer der vorhergehenden Aussagen, enthaltend
   a) einen oder mehrere Komplexbildner gemäß Formel (I) in einem Gewichtsanteil von 0,001-10 Gew.-% am Gesamtgewicht der Zusammensetzung und
   b) ein oder mehrere Antioxidantien aus der Gruppe der Gruppe der Ascorbinsäure, Ascorbinsäuresalze, Ascorbylester oder Mischungen davon in einem Gewichtsanteil von 0,0005-1 Gew.-% am Gesamtgewicht der Zusammensetzung.
4. Stabilisierte Zusammensetzung nach einer der vorhergehenden Aussagen, enthaltend mindestens einen Komplexbildner (a) der allgemeinen Formel (I), wobei
   - R1 für ein Wasserstoffatom, eine lineare oder verzweigte Carboxy-C₁-C₆-Alkylgruppe oder für ein physiologisch verträgliches Salz hiervon steht, bevorzugt für ein Wasserstoffatom, eine Carboxymethylgruppe oder für ein physiologisch verträgliches Salz hiervon oder für eine Gruppe -CH(CH₃)-COOH oder für ein physiologisch verträgliches Salz hiervon, steht.
5. Stabilisierte Zusammensetzung nach einer der vorhergehenden Aussagen, enthaltend mindestens einen Komplexbildner (a) der allgemeinen Formel (I), wobei
   - R2, R3 unabhängig voneinander für ein Wasserstoffatom, eine Methylgruppe, eine Carboxymethylgruppe oder für ein physiologisch verträgliches Salz hiervon oder für eine Gruppe -CH(CH₃)-COOH oder für ein physiologisch verträgliches Salz hiervon stehen.
6. Stabilisierte Zusammensetzung nach einer der Aussagen 1 bis 4, enthaltend mindestens einen Komplexbildner (a) der allgemeinen Formel (I), wobei
   - R1 für eine Carboxymethylgruppe oder für ein physiologisch verträgliches Salz hiervon steht,
   - R2 für eine Methylgruppe steht und
   - R3 für ein Wasserstoffatom steht.
7. Stabilisierte Zusammensetzung nach einer der Aussagen 1 bis 4, enthaltend mindestens einen Komplexbildner (a) der allgemeinen Formel (I), wobei
   - R1 für ein Wasserstoffatom steht,
   - R2, R3 unabhängig voneinander für eine Carboxymethylgruppe oder für ein physiologisch verträgliches Salz hiervon stehen.
8. Stabilisierte Zusammensetzung nach einer der Aussagen 1 bis 4, enthaltend mindestens einen Komplexbildner (a) der allgemeinen Formel (I), wobei
   - R1 für eine Gruppe -CH(CH₃)-COOH oder für ein physiologisch verträgliches Salz hiervon steht,
   - R2, R3 unabhängig voneinander für eine Carboxymethylgruppe oder für ein physiologisch verträgliches Salz hiervon stehen.
9. Stabilisierte Zusammensetzung nach einer der vorhergehenden Aussagen, enthaltend Ascorbinsäure oder ein Alkali-, Ammonium-, Erdalkalisalz der Ascorbinsäure.
10. Stabilisierte Zusammensetzung nach Aussage 9, enthaltend Natriumhydroascorbat, Natriumascorbat, Kaliumhydroascorbat, Kaliumascorbat, Ammoniumhydroascorbat, Ammoniumascorbat, Calciumascorbat, Calciumhydroascorbat, Magnesiumascorbat, Magnesiumhydroascorbat oder Mischungen davon.
11. Stabilisierte Zusammensetzung nach einer der Aussagen 1 bis 8, enthaltend mindestens einen Ester der Ascorbinsäure mit einer organischen Säure.
12. Stabilisierte Zusammensetzung nach Aussage 11, enthaltend Ascorbylpalmitat, Ascorbylisopalmitat, Ascorbyltetraisopalmitat, Ascorbylstearat, Ascorbylisostearat, Ascorbyloleat, Ascorbyllinoleat oder Mischungen davon.
13. Stabilisierte Zusammensetzung nach einer der Aussagen 1 bis 8, enthaltend mindestens einen Ester der Ascorbinsäure mit einer anorganischen Säure oder einem Salz davon.
14. Stabilisierte Zusammensetzung nach Aussage 13, enthaltend mindestens einen Ester der Ascorbinsäure mit Phosphorsäure oder einem Salz davon.
15. Stabilisierte Zusammensetzung nach einer der Aussagen 13 oder 14, enthaltend Sodium Ascorbyl Phosphate, Magnesium Ascorbyl Phosphate oder Mischungen davon.
16. Stabilisierte Zusammensetzung nach einer der Aussagen 13 bis 15, enthaltend Sodium Ascorbyl Phosphate in einem Gewichtsanteil von 0,01-0,5 Gew.-% am Gesamtgewicht der Zusammensetzung.
17. Stabilisierte Zusammensetzung nach einer der Aussagen 1 bis 16, wobei die Zusammensetzung eine kosmetische Zusammensetzung ist.
18. Stabilisierte Zusammensetzung nach einer der vorhergehenden Aussagen, enthaltend mindestens ein Tensid in einem Gewichtsanteil von 0,5-25 Gew.-% am Gesamtgewicht der Zusammensetzung.
19. Stabilisierte Zusammensetzung nach einer der vorhergehenden Aussagen, enthaltend mindestens einen Farbstoff.
20. Stabilisierte Zusammensetzung nach einer der vorhergehenden Aussagen, enthaltend mindestens einen Duftstoff.
21. Stabilisierte kosmetische Zusammensetzung nach einer der vorhergehenden Aussagen in Form einer Reinigungszubereitung für Haut oder Haare.
22. Stabilisierte kosmetische Zusammensetzung nach Aussage 21, enthaltend mindestens ein anionisches Tensid, amphoteres Tensid, zwitterionisches Tensid oder Mischungen davon in einem Gewichtsanteil von 1-20 Gew.-% am Gesamtgewicht der Zusammensetzung.
23. Stabilisierte kosmetische Zusammensetzung nach einer der Aussagen 1 bis 20 in Form eines Rinse-off oder Leave-on-Haarpflegezusammensetzung.
24. Stabilisierte kosmetische Zusammensetzung nach Aussage 23, enthaltend mindestens ein kationisches Tensid in einem Gewichtsanteil von 0,1-10 Gew.-% am Gesamtgewicht der Zusammensetzung.
25. Stabilisierte kosmetische Zusammensetzung nach einerder Aussagen 1 bis 24, enthaltend eine oder mehrere Verbindungen aus der Gruppe der kationischen Polymere, der natürlichen, mineralischen oder synthetischen Öl-, Fett- oder Wachskomponenten.
26. Stabilisierte kosmetische Zusammensetzung nach einer der Aussagen 1 bis 20 in Form eines Hautpflegeprodukts, Enthaarungsmittels, Rasierhilfsmittels, Sonnenschutzmittels, After-Sun-Präparats, Make-ups, Wimperntusche, Nagellacks, Dauerwellmittels, Entkräuselungsmittels, Haarsprays, Haargels, Haarwachses, eines permanenten, semipermanenten oder temporären Haarfärbemittels.
27. Verwendung
   a) mindestens eines Komplexbildners der allgemeinen Formel (I) 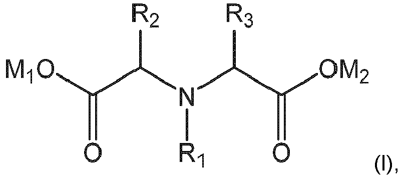 wobei
      - R1 für ein Wasserstoffatom, eine lineare oder verzweigte C₁-C₆-Alkylgruppe, eine lineare oder verzweigte Hydroxy-C₂-C₆-alkylgruppe, eine lineare oder verzweigte Carboxy-C₁-C₆-Alkylgruppe oder für ein physiologisch verträgliches Salz hiervon steht,
      - R2, R3 unabhängig voneinander für ein Wasserstoffatom, eine lineare oder verzweigte C₁-C₆-Alkylgruppe, eine lineare oder verzweigte Hydroxy-C₂-C₆-alkylgruppe, eine lineare oder verzweigte Carboxy-C₁-C₆-Alkylgruppe oder für ein physiologisch verträgliches Salz hiervon stehen,
      - M1, M2 unabhängig voneinander für ein Wasserstoffatom oder ein Äquivalent eines Alkali-, Erdalkali- oder Metallions, bevorzugt für Natrium, Kalium, ½ Magnesium, ½ Calcium, ½ Zink, oder für ein Ammoniumion (NH₄⁺) stehen, und
   b) mindestens eines Antioxidans, ausgewählt aus der Gruppe der Ascorbinsäure, Ascorbinsäuresalze, Ascorbylester oder Mischungen davon
   zur Stabilisierung von Haushaltsprodukten, kosmetischen oder pharmazeutischen Zusammensetzungen gegen die schädlichen Auswirkungen von Licht, Hitze und/oder Sauerstoff.
28. Verwendung nach Aussage 27 zur Stabilisierung Farbstoffe enthaltender Haushaltsprodukte, kosmetischer oder pharmazeutischer Zusammensetzungen gegen Farbveränderungen und/oder Entfärbungen.
29. Verwendung nach Aussage 27 zur Stabilisierung Farbstoff-freier Haushaltsprodukte, kosmetischer oder pharmazeutischer Zusammensetzungen gegen Verfärbungen.
30. Verfahren zur Stabilisierung von Haushaltsprodukten, kosmetischen oder pharmazeutischen Zusammensetzungen gegen die schädlichen Auswirkungen von Licht, Hitze und/oder Sauerstoff, dadurch gekennzeichnet, dass den Zusammensetzungen
   a) mindestens ein Komplexbildner der allgemeinen Formel (I) 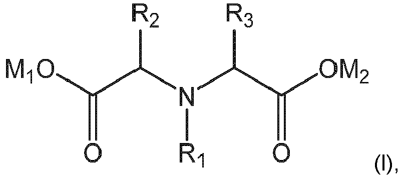 wobei
      - R1 für ein Wasserstoffatom, eine lineare oder verzweigte C₁-C₆-Alkylgruppe, eine lineare oder verzweigte Hydroxy-C₂-C₆-alkylgruppe, eine lineare oder verzweigte Carboxy-C₁-C₆-Alkylgruppe oder für ein physiologisch verträgliches Salz hiervon steht,
      - R2, R3 unabhängig voneinander für ein Wasserstoffatom, eine lineare oder verzweigte C₁-C₆-Alkylgruppe, eine lineare oder verzweigte Hydroxy-C₂-C₆-alkylgruppe, eine lineare oder verzweigte Carboxy-C₁-C₆-Alkylgruppe oder für ein physiologisch verträgliches Salz hiervon stehen,
      - M1, M2 unabhängig voneinander für ein Wasserstoffatom oder ein Äquivalent eines Alkali-, Erdalkali- oder Metallions, bevorzugt für Natrium, Kalium, ½ Magnesium, ½ Calcium, ½ Zink, oder für ein Ammoniumion (NH₄⁺) stehen, und
   b) mindestens ein Antioxidans, ausgewählt aus der Gruppe der Ascorbinsäure, Ascorbinsäuresalze, Ascorbylester oder Mischungen davon
   hinzugefügt wird.

Unter erfindungsgemäß geeigneten Zusammensetzungen werden vorzugsweise solche verstanden, die Wasser als Träger enthalten. Der Gewichtsanteil von Wasser am Gesamtgewicht der Zusammensetzungen beträgt vorzugsweise 5-90 Gew.-%, mehr bevorzugt 10-90 Gew.-%, besonders bevorzugt 25-90 Gew.-%, ganz besonders bevorzugt 40-90 Gew.-% und insbesondere 50-90 Gew.-%.

Es wurde gefunden, dass für eine ausgezeichnete Stabilisierungswirkung erfindungsgemäßer Zusammensetzungen - insbesondere gegenüber Farb- oder Geruchsveränderungen - bereits sehr geringe Mengen mindestens eines Antioxidans b) in Kombination mit mindestens einem spezifischen Komplexierungsmittel a) gemäß Formel (I) ausreichend sind.

Bevorzugte erfindungsgemäße Zusammensetzungen enthalten daher
a) einen oder mehrere Komplexbildner gemäß Formel (I) in einem Gewichtsanteil von 0,001-10 Gew.-%, vorzugsweise 0,005- 7,5 Gew.-%, mehr bevorzugt 0,01 - 5 Gew.-%, besonders bevorzugt 0,05 - 3 Gew.-% und insbesondere 0,1 - 2 Gew.-% am Gesamtgewicht der Zusammensetzung und
b) ein oder mehrere Antioxidantien aus der Gruppe der Gruppe der Ascorbinsäure, Ascorbinsäuresalze, Ascorbylester oder Mischungen davon in einem Gewichtsanteil von 0,0005-1 Gew.-%, vorzugsweise 0,001 - 0,9 Gew.-%, mehr bevorzugt 0,0025 - 0,75 Gew.-%, besonders bevorzugt 0,005 - 0,6 Gew.-% und insbesondere 0,01 - 0,5 Gew.-% am Gesamtgewicht der Zusammensetzung.

Komplexbildner a) der Formel (I) werden als biologisch abbaubar beschrieben und stellen daher einen ökologisch vorteilhaften Ersatz für HEDP oder EDTA dar. Überraschenderweise hat sich herausgestellt, dass zudem mit Einsatz dieser speziellen Komplexbildner a) gemäß Formel (I) sowie mindestens einem Antioxidans b) in erfindungsgemäßen Mitteln eine besonders gute Stabilisierungswirkung gegenüber Farb- und Geruchsveränderungen erzielt werden kann.

Im Folgenden werden Beispiele für die in Formel (I) genannten Substituenten R1, R2 und R3 exemplarisch genannt: Beispiele für C₁-C₆-Alkylreste sind -CH₃, -CH₂CH₃, -CH₂CH₂CH₃, -CH(CH₃)₂, -CH₂CH(CH₃)₂, -CH(CH₃)CH₂CH₃, -C(CH₃)₃, -(CH₂)₄CH₃, -(CH₂)₅CH₃. Besonders bevorzugte Alkylreste sind Methyl und Ethyl.

Beispiele für C₁-C₆-Hydroxyalkylgruppen sind -CH₂OH, -CH₂CH₂OH, -CH₂CH₂CH₂OH, -CH₂CH(OH)CH₃, -CH₂CH₂CH₂CH₂OH, wobei -CH₂CH₂OH bevorzugt ist.

Beispiele für Carboxy-C₁-C₆-Alkylgruppen sind HOOC-CH₂-, HOOC-CH₂-CH₂-, HOOC-CH₂-CH₂-CH₂-, HOOC-CH₂-CH₂-CH₂-CH₂-, HOOC-CH(CH₃)-, HOOC-C(CH₃)₂-, HOOC-CH(CH₃)-CH₂-, HOOC-CH₂-CH(CH₃)-, wobei die Gruppen HOOC-CH₂- und HOOC-CH(CH₃)-bevorzugt sind.

Unter physiologisch verträglichen Salzen werden die Salze verstanden, die unter physiologischen Bedingungen ohne nachteilige Wirkung in kosmetischen oder pharmazeutischen Produkten eingesetzt werden können. Beispiele für ein physiologisch verträgliches Salz einer Carboxy-C₁-C₆ - Alkylgruppe sind beispielsweise das Natriumsalz, das Kaliumsalz und das Ammoniumsalz der Carboxy-C₁-C₆-Alkylgruppe.

Besonders gute Stabilisierungsergebnisse konnten mit erfindungsgemäßen Zusammensetzungen beobachtet werden, die mindestens einen Komplexbilder a) der allgemeinen Formel (I) enthielten, wobei der Rest
- R1: für ein Wasserstoffatom, eine lineare oder verzweigte Carboxy-C₁-C₆-Alkylgruppe oder für ein physiologisch verträgliches Salz hiervon steht, bevorzugt für ein Wasserstoffatom, eine Carboxymethylgruppe oder für ein physiologisch verträgliches Salz hiervon oder für eine Gruppe -CH(CH₃)-COOH oder für ein physiologisch verträgliches Salz hiervon, steht.

Weiterhin besonders gute Stabilisierungsergebnisse konnten mit erfindungsgemäßen Zusammensetzungen beobachtet werden, die mindestens einen Komplexbilder a) der allgemeinen Formel (I) enthielten, wobei die Reste
- R2, R3: unabhängig voneinander für ein Wasserstoffatom, eine Methylgruppe, eine Carboxymethylgruppe oder für ein physiologisch verträgliches Salz hiervon oder für eine Gruppe -CH(CH₃)-COOH oder für ein physiologisch verträgliches Salz hiervon stehen.

Ganz besonders gute Stabilisierungsergebnisse konnten mit erfindungsgemäßen Zusammensetzungen beobachtet werden, die mindestens einen Komplexbilder a) der allgemeinen Formel (I) enthielten, worin
(i) R1 für eine Carboxymethylgruppe oder für ein physiologisch verträgliches Salz hiervon steht; R2 für eine Methylgruppe steht und R3 für ein Wasserstoffatom steht, oder
(ii) R1 für ein Wasserstoffatom steht und R2, R3 unabhängig voneinander für eine Carboxymethylgruppe oder für ein physiologisch verträgliches Salz hiervon stehen, oder
(iii) R1 für eine Gruppe -CH(CH₃)-COOH oder für ein physiologisch verträgliches Salz hiervon steht und R2, R3 unabhängig voneinander für eine Carboxymethylgruppe oder für ein physiologisch verträgliches Salz hiervon stehen.

Ein erfindungsgemäß ganz besonders bevorzugter Komplexbildner a) ist N,N-Bis(carboxymethyl)-L-alanin, das alternativ auch als 2-Methyl-2',2",2"'-nitrilotriessigsäure oder Methylglycindiacetic acid bezeichnet werden kann und als Substanz MGDA abgekürzt wird. MGDA besitzt die CAS-Nummer 29578-05-0 und kann kommerziell von verschiedenen Anbietern, beispielsweise von der Firma ABClabtory Scientific Co. Ltd, von der Firma Chemieliva Pharmaceutical Co. Ltd., von der Firma SIA "Chemspace" oder von der Firma Hong Kong Chemhere Co. Ltd, erworben werden. MGDA hat die Formel (I-a)

HO-C(O)-CH(CH₃)-N(CH₂COOH)₂ (I-a).

Auch die physiologisch verträglichen Salze von MGDA - wie insbesondere das Trinatriumsalz des MGDA - sind erfindungsgemäß geeignet und besonders bevorzugt.

Ein weiterer erfindungsgemäß ganz besonders bevorzugter Komplexbildner a) ist Aspartic acid-N-(2,3-dicarboxyethyl)tetranatriumsalz, das alternativ auch als Tetranatriumiminodissucinat bezeichnet werden kann und die CAS-Nummer 144538-83-0 trägt. Unter dem Handelsnamen Baypure CX 100 ist dieser Komplexbildner von der Firma Lanxess kommerziell zu erwerben.

Ein weiterer geeigneter Komplexbildner a) der Formel (I) ist Nitrilotriessigsäure (NTA). Bei Nitrilotriessigsäure steht der Rest R1 für eine Carboxymethylgruppe oder für ein physiologisch verträgliches Salz hievon, und R2, R3 stehen beide für ein Wasserstoffatom.

Antioxidantien b) im Sinne der vorliegenden Erfindung sind Ascorbinsäure, Ascorbinsäuresalze, Ascorbylester oder Mischungen davon.

Unter geeigneten Salzen der Ascorbinsäure werden Alkali-, Ammonium- oder Erdalkalisalze der Ascorbinsäure verstanden, wie beispielsweise Natriumhydroascorbat, Natriumascorbat, Kaliumhydroascorbat, Kaliumascorbat, Ammoniumhydroascorbat, Ammoniumascorbat, Calciumascorbat, Calciumhydroascorbat, Magnesiumascorbat, Magnesiumhydroascorbat oder Mischungen davon.

Geeignete Ascorbinsäureester im Sinne der vorliegenden Erfindung können bevorzugt ausgewählt sein aus Estern von Ascorbinsäure mit organischen Säuren gemäß der nachfolgenden Formel (II) 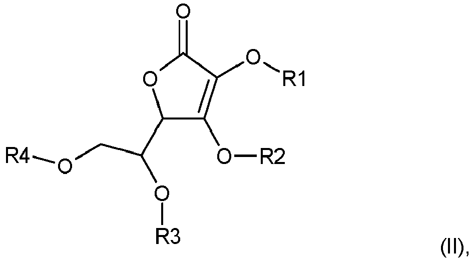 in der
einer bis vier der Reste R1 bis R4 für die Gruppierung -C(O)-R und die anderen Reste ggfs. für Wasserstoff stehen; und R für einen geradkettigen oder verzweigten, gesättigten oder
ungesättigten Alk(en)ylrest mit 8 bis 24, bevorzugt mit 10 bis 20 und insbesondere mit 13 bis 17 Kohlenstoffatomen steht.

Bevorzugte Ester von Ascorbinsäure mit organischen Säuren sind die folgenden Verbindungen: Ascorbylpalmitat, Ascorbylisopalmitat, Ascorbyltetraisopalmitat, Ascorbylstearat, Ascorbylisostearat, Ascorbyloleat, Ascorbyllinoleat oder Mischungen davon.

Weiterhin geeignete Ascorbinsäureester im Sinne der vorliegenden Erfindung sind Ester von Ascorbinsäure mit anorganischen Säuren, bevorzugt mit Phosphorsäure oder Schwefelsäure. Besonders bevorzugt sind Ascorbylphosphat und/oder Ascorbylsulfat bzw. die Alkali- oder Erdalkalisalze des Ascorbylphosphats und/oder Ascorbylsulfats, wie beispielsweise die unter den INCI-Bezeichnungen Sodium Ascorbyl Phosphate, Magnesium Ascorbyl Phosphate, Sodium Ascorbyl Sulfate und/oder Magnesium Ascorbyl Sulfate bekannten Verbindungen. Sodium Ascorbyl Phosphate ist aufgrund seiner ausgezeichneten Stabilität in wässrigen Medien ein insbesondere bevorzugter anorganischer Ascorbinsäureester für die Verwendung als Antioxidans b) in den erfindungsgemäßen Zusammensetzungen.

Ganz besonders bevorzugte erfindungsgemäße Zusammensetzungen enthalten daher Sodium Ascorbyl Phosphate in einem Gewichtsanteil von 0,01-0,5 Gew.-% am Gesamtgewicht der Zusammensetzung.

Besonders bevorzugte Ausführungsformen erfindungsgemäßer Zusammensetzungen sind folgendermaßen gekennzeichnet (Mengenangaben in Gew.-%):

| | **1** | **2** | **3** | **4** | **5** |
|---|---|---|---|---|---|
| Komplexbildner gemäß Formel (I)*¹ | 0,001-10 | 0,005-7,5 | 0,01-5 | 0,05-3 | 0,1-2 |
| Ascorbinsäure und/oder Ascorbinsäuresalz*² | 0,0005-1 | 0,001-0,9 | 0,0025-0,75 | 0,005-0,6 | 0,01-0,5 |

| | | | | | |
|---|---|---|---|---|---|
| *¹ MGDA (oder Trinatriumsalz der MGDA) und/oder Tetranatriumiminodissucinat und/oder Nitrilotriessigsäure (NTA) *² Natriumhydroascorbat, Natriumascorbat, Kaliumhydroascorbat, Kaliumascorbat, Ammoniumhydroascorbat, Ammoniumascorbat, Calciumascorbat, Calciumhydroascorbat, Magnesiumascorbat und/oder Magnesiumhydroascorbat | | | | | |

| | **6** | **7** | **8** | **9** | **10** |
|---|---|---|---|---|---|
| Komplex-bildner gemäß Formel (I)*¹ | 0,001-10 | 0,005-7,5 | 0,01-5 | 0,05-3 | 0,1-2 |
| Ester von Ascorbinsäure und einer organischen Säure bzw. Salz davon*³ | 0,0005-1 | 0,001-0,9 | 0,0025-0,75 | 0,005-0,6 | 0,01-0,5 |

| | | | | | |
|---|---|---|---|---|---|
| *3 Ascorbylpalmitat, Ascorbylisopalmitat, Ascorbyltetraisopalmitat, Ascorbylstearat, Ascorbylisostearat, Ascorbyloleat und/oder Ascorbyllinoleat | | | | | |

| | **11** | **12** | **13** | **14** | **15** |
|---|---|---|---|---|---|
| Komplex-bildner gemäß Formel (I)*¹ | 0,001-10 | 0,005-7,5 | 0,01-5 | 0,05-3 | 0,1-2 |
| Ester von Ascorbinsäure und einer anorganischen Säure bzw. Salz davon*⁴ | 0,0005-1 | 0,001-0,9 | 0,0025-0,75 | 0,005-0,6 | 0,01-0,5 |

| | | | | | |
|---|---|---|---|---|---|
| *⁴ Sodium Ascorbyl Phosphate, Magnesium Ascorbyl Phosphate, Sodium Ascorbyl Sulfate und/oder Magnesium Ascorbyl Sulfate | | | | | |

Ganz besonders bevorzugte stabilisierte Zusammensetzungen im Sinne der vorliegenden Erfindung enthalten (bezogen auf ihr Gesamtgewicht)
a) 0,001-10 Gew.-%, vorzugsweise 0,005 - 7,5 Gew.-%, mehr bevorzugt 0,01 - 5 Gew.-%, besonders bevorzugt 0,05 - 3 Gew.-% und insbesondere 0,1 - 2 Gew.-% MGDA (oder Trinatriumsalz der MGDA) sowie
b) 0,0005-1 Gew.-%, vorzugsweise 0,001 - 0,9 Gew.-%, mehr bevorzugt 0,0025 - 0,75 Gew.-%, besonders bevorzugt 0,005 - 0,6 Gew.-% und insbesondere 0,01 - 0,5 Gew.-% Sodium Ascorbyl Phosphate.

Innerhalb dieser Ausführungsform sind erfindungsgemäße Zusammensetzungen weiterhin bevorzugt, die (bezogen auf ihr Gesamtgewicht)
c) 5-90 Gew.-% Wasser und
d) gegebenenfalls bis zu 1 Gew.-% mindestens eines Farbstoffs und/oder mindestens eines Duftstoffs enthalten.

In einer ersten ganz besonders bevorzugten Ausführungsform handelt es sich bei erfindungsgemäßen Zusammensetzungen um kosmetische Zusammensetzungen.

Kosmetische Zusammensetzungen im Sinne der vorliegenden Erfindung enthalten das bereits beschriebene Stabilisierungssystem a)+b) vorzugsweise in einem kosmetischen Träger.

In einer besonders bevorzugten Ausführungsform ist der kosmetische Träger wässrig.

Ein wässriger kosmetischer Träger bedeutet im Sinne der vorliegenden Erfindung, dass die kosmetische Zusammensetzung vorzugsweise mindestens 50 Gew.-%, mehr bevorzugt mindestens 60 Gew.-% und insbesondere bevorzugt mindestens 70 Gew.-% Wasser enthält (bezogen auf das Gesamtgewicht der kosmetischen Zusammensetzung).

Daneben enthalten kosmetische Zusammensetzungen im Sinne der vorliegenden Erfindung vorzugsweise
- mindestens ein Tensid in einem Gewichtsanteil von 0,5-25 Gew.-% am Gesamtgewicht der Zusammensetzung sowie
- gegebenenfalls mindestens einen Farbstoff und/oder mindestens einen Duftstoff in einem Gewichtsanteil von bis zu 1 Gew.-% am Gesamtgewicht der kosmetischen Zusammensetzung.

Innerhalb dieser ersten ganz besonders bevorzugten Ausführungsform sind unter kosmetischen Zusammensetzungen vorzugsweise Reinigungszusammensetzungen für Haut oder Haare zu verstehen, die neben dem Stabilisierungssystem a)+b) in einem kosmetischen Träger (s.o.) mindestens ein anionisches Tensid, amphoteres Tensid, zwitterionisches Tensid oder Mischungen davon in einem Gewichtsanteil von 1-20 Gew.-% am Gesamtgewicht der Reinigungszusammensetzung enthalten.

Unter Reinigungszusammensetzungen für die Haut und/oder die Haare sind beispielsweise Haarshampoos, haarkonditionierende Shampoos, Haarspülungen, Haarfärbeshampoos, Duschgele, Duschbäder, Schaumbäder oder dergleichen zu verstehen.

Vorzugsweise handelt es sich bei den kosmetischen Reinigungszusammensetzungen um Haarshampoos oder Duschzusammensetzungen.

Geeignete Reinigungszusammensetzungen im Sinne der vorliegenden Erfindung können beispielsweise als O/W-, W/O-, W/O/W- Emulsionen in Form von Cremes oder Gelen, als schäumende Lösungen oder andere Zubereitungen vorliegen, die für die Anwendung auf der Haut oder dem Haar geeignet sind.

Geeignete Reinigungszusammensetzungen im Sinne der vorliegenden Erfindung weisen vorzugsweise einen pH-Wert im Bereich von etwa 3 - 6, mehr bevorzugt 3,5 - 5,5 und insbesondere 4 - 5 auf.

Innerhalb dieser ersten besonders bevorzugten Ausführungsform sind unter kosmetischen Zusammensetzungen vorzugsweise auch Rinse-off oder Leave-on-Haarpflegezusammensetzungen zu verstehen, die neben dem Stabilisierungssystem a)+b) in einem kosmetischen Träger (s.o.) mindestens ein kationisches Tensid in einem Gewichtsanteil von 0,1-10 Gew.-% am Gesamtgewicht der Zusammensetzung enthalten.

Unter Rinse-off oder Leave-on-Haarpflegezusammensetzungen sind beispielsweise Haarspülungen, Haarkuren, Seren, Tonics, Haarcremes oder dergleichen zu verstehen.

Geeignete Rinse-off oder Leave-on-Haarpflegezusammensetzungen im Sinne der vorliegenden Erfindung können beispielsweise als O/W-, W/O-, W/O/W- Emulsionen in Form von Cremes oder Gelen, als schäumende Lösungen oder andere Zubereitungen vorliegen, die für die Anwendung auf dem Haar geeignet sind.

Geeignete Rinse-off oder Leave-on-Haarpflegezusammensetzungen im Sinne der vorliegenden Erfindung weisen vorzugsweise einen pH-Wert im Bereich von etwa 2 - 5, mehr bevorzugt 2,5 - 4,5 und insbesondere 3 - 4 auf.

Unter geeigneten anionischen Tensiden, die vorzugsweise in erfindungsgemäßen Reinigungszusammensetzungen eingesetzt werden können, sind beispielsweise Vertreter aus den folgenden Gruppen sowie deren physiologisch verträgliche Salze zu verstehen:
- lineare und verzweigte Fettsäuren mit 8 bis 30 C-Atomen (Seifen),
- Ethercarbonsäuren der Formel R-0-(CH₂-CH₂O)x-CH₂-COOH, in der R eine lineare oder verzweigte, gesättigte oder ungesättigte Alkylgruppe mit 8 bis 30 C-Atomen und x = 0 oder 1 bis 16 ist,
- Acylaminosäuren mit 8 bis 24 C-Atomen in der Acylgruppe,
- Acyltauride mit 8 bis 24 C-Atomen in der Acylgruppe (Taurat-Tenside),
- Acylisethionate mit 8 bis 24 C-Atomen in der Acylgruppe (Isethionat-Tenside),
- Sulfobernsteinsäuremono- und/oder-dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen (Sulfosuccinat-Tenside),
- Alpha-Olefinsulfonate mit 8 bis 24 C-Atomen (alpha-Olefinsulfonat-Tenside),
- Alkylsulfate und/oder Alkylethersulfatsalze der Formel R-(OCH2-CH2)n-O-SO3X, in der R bevorzugt eine geradkettige oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen, und/oder
- Alkyl- und/oder Alkenyletherphosphate der Formel 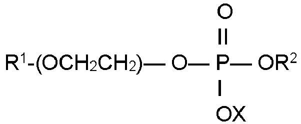 in der R¹ bevorzugt für einen aliphatischen Kohlenwasserstoffrest mit 8 bis 30 Kohlenstoffatomen, R² für Wasserstoff, einen Rest (CH₂CH₂0)ₙR¹ oder X, n für Zahlen von 0 bis 10 und Xfür Wasserstoff, ein Alkali- oder Erdalkalimetall oder die Gruppe - NR³R⁴R⁵R⁶ steht, mit R³ bis R⁶ unabhängig voneinander stehend für einen Ci bis C₄ - Kohlenwasserstoffrest.

Besonders bevorzugt sind Alkylsulfat- und/oder Alkylethersulfatsalze, (Salze von) Ethercarbonsäuren, Sarcosinaten, Isethionaten, Tauraten, Sulfosuccinaten und/oder Alpha-Olefinsulfonaten. Insbesondere bevorzugt sind Alkylsulfat- und/oder Alkylethersulfatsalze.

Das oder die anionische Tensid(e) kann (können) in erfindungsgemäßen Reinigungsmitteln (bezogen auf das Gewicht des gesamten Mittels) bevorzugt in einer Menge von 2,5 bis 20 Gew.-%, mehr bevorzugt von 3 bis 18 Gew.-%, besonders bevorzugt von 4 bis 16 Gew.-%, ganz besonders bevorzugt von 5 bis 14 Gew.-% und insbesondere von 6 bis 12,5 Gew.-% eingesetzt werden.

Zur weiteren Stabilisierung der Schaumeigenschaften und Hautmilde erfindungsgemäßer Reinigungszusammensetzungen kann es von Vorteil, wenn neben anionischen Tensiden zudem hautverträgliche, gut schäumbare amphotere, zwitterionische, nichtionische Tenside oder Mischungen davon eingesetzt werden.

Zwitterionische und/oder amphotere (ampholytische) Tenside können in den erfindungsgemäßen Reinigungsmitteln vorzugsweise in Mengen von 0 - 10 Gew.-%, mehr bevorzugt von 0,5 - 9 Gew.-% und insbesondere von 1 - 7,5 Gew.-% (bezogen auf das Gesamtgewicht des Reinigungsmittels) eingesetzt werden.

Nichtionische Tenside können in den erfindungsgemäßen Reinigungsmitteln vorzugsweise in Mengen von 0 - 10 Gew.-%, mehr bevorzugt von 0,1 - 7,5 Gew.-% und insbesondere von 0,5 - 5 Gew.-% (bezogen auf das Gesamtgewicht des Reinigungsmittels) eingesetzt werden.

Unter erfindungsgemäß geeigneten zwitterionischen Tensiden sind die sogenannte Betaine wie Alkylbetaine, N-Alkyl-N,N-dimethylammoniumglycinate, beispielsweise das Kokosalkyldimethylammoniumglycinat, N-Acyl-aminopropyl-N,N-dimethylammoniumglycinate, beispielsweise Kokosamidopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie Kokosaminoethylhydroxyethylcarboxymethylglycinat zu verstehen. Ein bevorzugtes zwitterionisches Tensid ist das unter der INCI-Bezeichnung Cocamidopropyl Betaine bekannte Fettsäureamid-Derivat.

Beispiele für erfindungsgemäß geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Bevorzugte ampholytische Tenside sind N-Kokosalkylaminopropionat, Kokosacylaminoethylaminopropionat und C₁₂-C₁₈-Acylsarcosin.

Besonders geeignete amphotere und/oder zwitterionische Tenside sind aufgrund ihrer ausgezeichneten Milde und guten Schaumeigenschaften Ampho- und Betaintenside, die eine Alkyl- bzw. Acylgruppe mit 8 bis 10 Kohlenstoffatomen aufweisen, insbesondere Betaintenside der Formel R²CONH(CH₂)ₘ-N⁺(CH₃)₂-COO⁻,in der R²CO eine von Capryl- und Caprinsäure abgeleitete Gruppe und m = 3 ist. Ein solches Tensid ist als besonders haut- und schleimhautfreundliches sowie reizarmes Betaintensid bekannt, und unter der INCI-Bezeichnung Cocamidopropyl Betaine beispielsweise von den Firmen BASF oder Evonik im Handel erhältlich.

Erfindungsgemäß geeignete nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- alkoxylierte Triglyceride,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine oder
- Zuckertenside vom Typ der Alkyl- und Alkenyloligoglykoside.

Besonders geeignete nichtionische Tenside sind aufgrund ihrer ausgezeichneten Milde Alkyl(oligo)glycoside.

In einer besonders bevorzugten Ausführungsform enthalten erfindungsgemäße Zusammensetzungen, die als Reinigungszusammensetzungen konfektioniert werden, in einem kosmetischen Träger
a) 0,001-10 Gew.-% mindestens eines Komplexbildners gemäß Formel (I),
b) 0,0005-1 Gew.-% mindestens eines Antioxidans, ausgewählt aus der Gruppe der Ascorbinsäure, Ascorbinsäuresalze, Ascorbylester oder Mischungen davon,
c) 2,5-20 Gew.-% mindestens eines anionischen Tensids,
d) 0-10 Gew.-% mindestens eines amphoteren und/oder zwitterionischen Tensids,
e) 0-10 Gew.-% mindestens eines nichtionischen Tensids,
f) 0-1 Gew.-% mindestens eines Farbstoffs und/oder mindestens eines Duftstoffs,
wobei sich die Mengenangaben auf das Gesamtgewicht der Reinigungszusammensetzungen beziehen.

Innerhalb dieser Ausführungsform sind Reinigungszusammensetzungen besonders bevorzugt, die in einem kosmetischen Träger
a) 0,001-10 Gew.-% MGDA oder das Trinatriumsalz des MGDA,
b) 0,0005-1 Gew.-% Sodium Ascorbyl Phosphate,
c) 2,5-20 Gew.-% mindestens eines Alkyl(ether)sulfats, insbesondere Sodium Laureth Sulfate,
d) 0-10 Gew.-% mindestens eines Betains, insbesondere Cocamidopropyl Betaine,
f) 0-1 Gew.-% mindestens eines Farbstoffs und/oder mindestens eines Duftstoffs enthalten,
wobei sich die Mengenangaben auf das Gesamtgewicht der Reinigungszusammensetzungen beziehen.

Unter geeigneten kationischen Tensiden, die vorzugsweise in erfindungsgemäßen Rinse-off oder Leave-on-Haarpflegezusammensetzungen eingesetzt werden können, sind beispielsweise:
- kationische Tenside vom Typ der quartären Ammoniumverbindungen,
- Esterquats und/oder
- Amidoamine.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride. Die langen Alkylketten dieser Tenside weisen bevorzugt 10 bis 18 Kohlenstoffatome auf, wie z. B. in Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere bevorzugte kationische Tenside sind die unter den INCI-Bezeichnungen Quaternium-27 und Quaternium-83 bekannten Imidazolium-Verbindungen.

Besonders bevorzugte erfindungsgemäße Zusammensetzungen, insbesondere Zusammensetzungen für die Pflege von Keratinfasern, enthalten - bezogen auf ihr Gewicht - vorzugsweise 0,05 bis 7,5 Gew.-%, besonders bevorzugt 0,1 bis 6 Gew.-% und insbesondere 0,25 bis 5 Gew.-% kationische(s) Tensid(e). Ganz besonders bevorzugt enthalten erfindungsgemäße Zusammensetzungen kationische Tenside aus der Gruppe der quartären Ammoniumverbindungen und/oder der Esterquats und/oder der Amidoamine in den zuvor genannten Mengen.

Kosmetische Zusammensetzungen im Sinne der vorliegenden Erfindung können zur Steigerung ihrer Pflegeeigenschaften vorzugsweise weiterhin mindestens ein Konditioniermittel enthalten.

In einer bevorzugten Ausführungsform enthalten erfindungsgemäße kosmetische Zusammensetzungen mindestens ein Konditioniermittel in einem Gewichtsanteil von 0 - 50 Gew.-% am Gesamtgewicht der kosmetischen Zusammensetzungen. Mehr bevorzugt sind Einsatzmengen von 0,01 - 30 Gew.-%, besonders bevorzugt von 0,05 - 25 Gew.-% und insbesondere von 0,1 - 20 Gew.-%.

Das mindestens eine Konditioniermittel kann vorzugsweise ausgewählt sein aus kationischen Polymeren, natürlichen, mineralischen oder synthetischen Ölen, Fetten oder Wachsen oder aus Mischungen davon.

Die erfindungsgemäßen kosmetischen Zusammensetzungen können mindestens ein kationisches Polymer enthalten. Vorzugsweise enthalten die erfindungsgemäßen kosmetischen Zusammensetzungen kationische Polysaccharid-Polymere.

Kationische Polysaccharid-Polymere steigern die Pflegeleistung der erfindungsgemäßen Mittel. Geeignete kationische Polysaccharid-Polymere können ausgewählt sein aus kationischen Celluloseverbindungen und/oder aus kationischen Guar-Derivaten.

Besonders bevorzugte erfindungsgemäße kosmetische Zusammensetzungen enthalten als kationische(s) Polysaccharid-Polymer(e) - bezogen auf das Gewicht der Zusammensetzungen - 0,01 bis 3 Gew.-%, besonders bevorzugt 0,05 bis 2 Gew.-% und insbesondere 0,1 bis 1,5 Gew.-% mindestens eines kationischen Polymers aus der Gruppe der kationischen Cellulosepolymere und/oder der kationischen Guarderivate.

Kationische Celluloseverbindungen im Sinne der Erfindung sind solche, die in mindestens einer Seitenkette mehr als eine permanente kationische Ladung tragen. Cellulose ist aus beta-1,4-glycosidisch-verknüpften D-Glucopyranose-Einheiten aufgebaut und bildet une, wasserunlösliche Ketten. Als "Seitenkette" einer Cellulose werden chemische Substituenten definiert, die an das Cellulosegerüst binden und nicht zur nativen Cellulose zählen, da sie z. B. durch chemische Synthese nachträglich eingeführt wurden.

Bevorzugt sind quaternisierte Cellulosepolymere, die aus Hydroxy(C₂-C₄)alkylcellulosen hervorgehen, besonders bevorzugt aus Hydroxyethylcellulosen.

Solche Polymere sind dem Fachmann bekannt und im Handel von verschiedenen Firmen erhältlich. Besonders bevorzugt sind die unter den INCI-Bezeichnungen Polyquaternium-4, Polyquaternium-10, Polyquaternium-24, Polyquaternium-67 und/oder Polyquaternium-72 bekannten kationischen Cellulosederivate. Ganz besonders bevorzugt sind Polyquaternium-10, Polyquaternium-24 und/oder Polyquaternium-67 und insbesondere bevorzugt ist Polyquaternium-10.

Bevorzugte erfindungsgemäße kosmetische Zusammensetzungen enthalten als kationische(s) Polysaccharid-Polymer(e) - bezogen auf das Gewicht des Mittels - 0,01 bis 3 Gew.-%, besonders bevorzugt 0,05 bis 2 Gew.-% und insbesondere 0,1 bis 1,5 Gew.-% mindestens eines Polymers aus der Gruppe der Polyquaternium-4, Polyquaternium-10, Polyquaternium-24, Polyquaternium-67 und/oder Polyquaternium-72.

Geeignete kationische Guar-Derivate im Sinne der Erfindung sind kationische Hydroxyalkyl-Guar-Derivate, vorzugsweise kationisches Hydroxyethyltrimethylammonium-Guar und/oder kationisches Hydroxypropyltrimethylammonium-Guar mit mittleren Molekulargewichten zwischen 100.000 und 2.000.000 Dalton. Insbesondere bevorzugt sind die unter der INCI-Bezeichnung Guar Hydroxypropyltrimonium Chloride und/oder Hydroxypropyl Guar Hydroxypropyltrimonium Chloride bekannten kationischen Guar-Polymere mit einem Molekulargewicht (Gewichtsmittel) zwischen 200.000 und 1.600.000 Dalton. Die kationische Ladungsdichte dieser Guar-Polymere beträgt vorzugsweise mindestens 0,4 meq/g, bevorzugt mindestens 0,5 meq/g und insbesondere mindestens 0,6 meq/g. Ihr Stickstoffgehalt liegt vorzugsweise im Bereich von 1,1 bis 1,8 Gew.-% (bezogen auf ihr Gesamtgewicht).

Kationische Guar-Derivate, die unter der INCI-Bezeichnung Guar Hydroxypropyltrimonium Chloride bekannt sind, sind dem Fachmann bekannt und beispielsweise unter den Handelsnamen Cosmedia^{®} Guar, N-Hance^{®} Jaguar^{®} und/oder Polycare^{®}von verschiedenen Anbietern erhältlich.

Besonders bevorzugte erfindungsgemäße Mittel enthalten als kationische(s) Polysaccharid-Polymere) - bezogen auf das Gewicht des Mittels - 0,01 bis 3 Gew.-%, besonders bevorzugt 0,05 bis 2 Gew.-% und insbesondere 0,1 bis 1,5 Gew.-% Guar Hydroxypropyltrimonium Chloride und/oder Hydroxypropyl Guar Hydroxypropyltrimonium Chloride.

Weitere geeignete kationische Polymere für die Verwendung in den erfindungsgemäßen Zusammensetzungen können beispielsweise ausgewählt sein aus kationischen Polymeren mir den INCI-Bezeichnungen Polyquaternium-2, Polyquaternium-5, Polyquaternium-6, Polyquaternium-7, Polyquaternium-11, Polyquaternium-17, Polyquaternium-18, Polyquaternium-22, Polyquaternium-27, Polyquaternium-37, Polyquaternium-39 sowie Mischungen davon.

Zusammenfassend sind erfindungsgemäß bevorzugte kosmetische Zusammensetzungen dadurch gekennzeichnet, dass sie - bezogen auf das Gewicht der Zusammensetzunghen - 0,01 bis 3 Gew.-%, besonders bevorzugt 0,05 bis 2 Gew.-% und insbesondere 0,1 bis 1,5 Gew.-% kationische(s) Polymer(e), vorzugsweise 0,01 bis 3 Gew.-%, besonders bevorzugt 0,05 bis 2 Gew.-% und insbesondere 0,1 bis 1,5 Gew.-% mindestens eines Polymers aus der Gruppe der kationischen Cellulosepolymeren und/oder der kationischen Guarderivate enthalten.

Die erfindungsgemäßen kosmetischen Zusammensetzungen können mindestens ein natürliches, mineralisches oder synthetisches Öl, Fett oder Wachs oder Mischungen davon enthalten.

Unter geeigneten natürlichen (pflanzlichen) Ölen werden üblicherweise Triglyceride und Mischungen von Triglyceriden verstanden. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Tamanuöl, Perillaöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaoabutter, Murumurubutter und/oder Shea-Butter verstanden.

Als mineralische Öle kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan (Cetiol^{®} S).

Als geeignete Ölkomponmente kann weiterhin ein Dialkylether dienen. Einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Din-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether. Besonders bevorzugt ist der Di-n-octylether, der im Handel unter der Bezeichnung Cetiol^{®} OE erhältlich ist.

Als synthetische Öle kommen bevorzugt Silikonverbindungen in Betracht. Silikone bewirken auf Keratinfasern, insbesondere auf Haaren, ausgezeichnete konditionierende Eigenschaften. Insbesondere bewirken sie eine bessere Kämmbarkeit von Haaren in nassem und trockenem Zustand und wirken sich in vielen Fällen positiv auf den Haargriff und die Weichheit der Haare aus. Geeignete Silikone können ausgewählt sein unter:
(i) Polyalkylsiloxanen, Polyarylsiloxanen, Polyalkylarylsiloxanen, die flüchtig oder nicht flüchtig, geradkettig, oder cyclisch, verzweigt oder nicht verzweigt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Unter Fetten sind zu verstehen Fettsäuren, Fettalkohole sowie natürliche und synthetische Wachse, welche sowohl in fester Form als auch flüssig in wässriger Dispersion vorliegen können.

Als Fettsäuren können eingesetzt werden lineare und/oder verzweigte, gesättigte und/oder ungesättigte Fettsäuren mit 6 - 30 Kohlenstoffatomen, bevorzugt mit 10 - 22 Kohlenstoffatomen. Als Fettalkohole können eingesetzt werden gesättigte, ein- oder mehrfach ungesättigte, e oder unverzweigte Fettalkohole mit C₆ - C₃₀-, bevorzugt C₁₀ - C₂₂- und ganz besonders bevorzugt C₁₂ - C₂₂- Kohlenstoffatomen.

Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, gehärtete Wachse wie beispielsweise Hydrogenated Castor Oil, Fruchtwachse wie beispielsweise Apfelwachs oder Citruswachs, Microwachse aus PE- oder PP.

Weitere geeignete Fettstoffe sind beispielsweise
- Esteröle, worunter Ester von C₆ - C₃₀ - Fettsäuren mit C₂ - C₃₀ - Fettalkohlen zu verstehen sind. Beispiele für besonders geeignete Esteröle sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetylpalmitat, Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V)
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat, symmetrische, unsymmetrische oder cyclische Ester der Kohlensäure mit Fettalkoholen wie Glycerincarbonat oder Dicaprylylcarbonat (Cetiol^{®} CC)
- ethoxylierte oder nicht ethoxylierte Mono,- Di- und Trifettsäureestervon gesättigten und/oder ungesättigten linearen und/oder en Fettsäuren mit Glycerin, wie beispielsweise Monomuls^{®} 90-O18, Monomuls^{®} 90-L12, Cetiol^{®} HE oder Cutina^{®} MD.

Der Gewichtsanteil der Öle, Fette und/oder Wachse am Gesamtgewicht der erfindungsgemäßen kosmetischen Zusammensetzungen beträgt vorzugsweise 0 bis 50 Gew.-%, besonders bevorzugt 0,01 bis 30 Gew.-% und insbesondere 0,05 bis 15 Gew.-%.

In weiteren bevorzugten Ausführungsformen können erfindungsgemäße kosmetische Zusammensetzungen zudem in Form eines(r) Hautpflegeprodukts, Enthaarungsmittels, Rasierhilfsmittels, Sonnenschutzmittels, After-Sun-Präparats, Make-ups, Wimperntusche, Nagellacks, Dauerwellmittels, Entkräuselungsmittels, Haarsprays, Haargels, Haarwachses, eines permanenten, semipermanenten oder temporären Haarfärbemittels konfektioniert werden und die darin üblichen Bestandteile in den üblichen Mengen enthalten.

Ein zweiter Gegenstand der Erfindung ist die Verwendung
a) mindestens eines Komplexbildners der allgemeinen Formel (I) 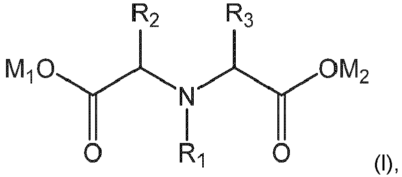 wobei
   - R1 für ein Wasserstoffatom, eine lineare oder verzweigte C₁-C₆-Alkylgruppe, eine lineare oder verzweigte Hydroxy-C₂-C₆-alkylgruppe, eine lineare oder verzweigte Carboxy-C₁-C₆-Alkylgruppe oder für ein physiologisch verträgliches Salz hiervon steht,
   - R2, R3 unabhängig voneinander für ein Wasserstoffatom, eine lineare oder verzweigte C₁-C₆-Alkylgruppe, eine lineare oder verzweigte Hydroxy-C₂-C₆-alkylgruppe, eine lineare oder verzweigte Carboxy-C₁-C₆-Alkylgruppe oder für ein physiologisch verträgliches Salz hiervon stehen,
   - M1, M2 unabhängig voneinander für ein Wasserstoffatom oder ein Äquivalent eines Alkali-, Erdalkali- oder Metallions, bevorzugt für Natrium, Kalium, ½ Magnesium, ½ Calcium, ½ Zink, oder für ein Ammoniumion (NH₄⁺) stehen, und
b) mindestens eines Antioxidans, ausgewählt aus der Gruppe der Ascorbinsäure, Ascorbinsäuresalze, Ascorbylester oder Mischungen davon
zur Stabilisierung von Haushaltsprodukten, kosmetischen oder pharmazeutischen Zusammensetzungen gegen die schädlichen Auswirkungen von Licht, Hitze und/oder Sauerstoff.

Unter "Stabilisierung von Haushaltsprodukten, kosmetischen oder pharmazeutischen Zusammensetzungen gegen die schädlichen Auswirkungen von Licht, Hitze und/oder Sauerstoff" ist vorzugsweise die Stabilisierung der vorgenannten Produkte gegen Farbveränderungen und/oder Entfärbungen zu verstehen.

Insbesondere ist darunter die Stabilisierung Farbstoff-freier Haushaltsprodukte, kosmetischer oder pharmazeutischer Zusammensetzungen gegen Verfärbungen und/oder die Stabilisierung Farbstoffenthaltender Haushaltsprodukte, kosmetischer oder pharmazeutischer Zusammensetzungen gegen Farbveränderungen oder Entfärbungen zu verstehen.

Ein dritter Gegenstand der Erfindung ist ein Verfahren zur Stabilisierung von Haushaltsprodukten, kosmetischen oder pharmazeutischen Zusammensetzungen gegen die schädlichen Auswirkungen von Licht, Hitze und/oder Sauerstoff, dadurch gekennzeichnet, dass den Zusammensetzungen
a) mindestens ein Komplexbildner der allgemeinen Formel (I) 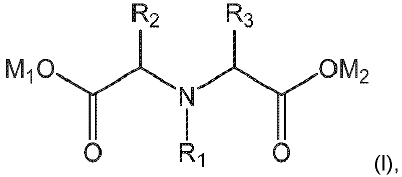 wobei
   - R1 für ein Wasserstoffatom, eine lineare oder verzweigte C₁-C₆-Alkylgruppe, eine lineare oder verzweigte Hydroxy-C₂-C₆-alkylgruppe, eine lineare oder verzweigte Carboxy-C₁-C₆-Alkylgruppe oder für ein physiologisch verträgliches Salz hiervon steht,
   - R2, R3 unabhängig voneinander für ein Wasserstoffatom, eine lineare oder verzweigte C₁-C₆-Alkylgruppe, eine lineare oder verzweigte Hydroxy-C₂-C₆-alkylgruppe, eine lineare oder verzweigte Carboxy-C₁-C₆-Alkylgruppe oder für ein physiologisch verträgliches Salz hiervon stehen,
   - M1, M2 unabhängig voneinander für ein Wasserstoffatom oder ein Äquivalent eines Alkali-, Erdalkali- oder Metallions, bevorzugt für Natrium, Kalium, ½ Magnesium, ½ Calcium, ½ Zink, oder für ein Ammoniumion (NH₄⁺) stehen, und
b) mindestens ein Antioxidans, ausgewählt aus der Gruppe der Ascorbinsäure, Ascorbinsäuresalze, Ascorbylester oder Mischungen davon
hinzugefügt wird.

### Beispiele

Die vorliegende Erfindung wird durch die folgenden nicht einschränkenden Beispiele veranschaulicht. Es sei darauf hingewiesen, dass verschiedene Änderungen und Modifikationen an den folgenden Beispielen vorgenommen werden können, ohne vom Umfang der Erfindung (welcher in den Patentansprüchen definiert ist) abzuweichen.

Daher sollte beachtet werden, dass die folgenden Beispiele nur als veranschaulichend und in keiner Weise einschränkend interpretiert werden sollten. Die folgende Tabelle umfasst Beispiele für kosmetische Reinigungszusammensetzungen (Mengenangaben in [Gew.-%]).

| | 1 | 2 | 3 | 4 | 5 |
|---|---|---|---|---|---|
| Komplexbildner a) gemäß Formel (I) | 0,001-10 | | | | |
| MGDA (oder MGDA-Trinatriumsalz) | | 0,005-7,5 | 0,01-5 | 0,05-3 | 0,1-2 |
| Ascorbinsäure, Ascorbinsäuresalz und/oder Ascorbylester | 0,0005-1 | | | | |
| Sodium Ascorbyl Phosphate | | 0,001-0,9 | 0,0025-0,75 | 0,005-0,6 | 0,01-0,5 |
| anionisches Tensid | 2,5-20 | | | | |
| Sodium Laureth Sulfate | | 3-18 | 4-16 | 5-14 | 6-12,5 |
| amphoteres oder zwitterionisches Tensid | 0-10 | | | | |
| Cocamidopropylbetain | | 0-10 | 0,5-9 | 1-7,5 | 1-7,5 |
| Öl, Fett und/oder Wachs | 0-50 | | | | |
| Mandelöl | | 0-30 | 0-20 | 0,01-5 | 0,01-5 |
| kationisches Polymer | 0-3 | | | | |
| Wasser und ggfs. weitere Inhaltsstoffe wie beispielsweise Konservierungsmittel, pH-Stellmittel, Parfum, Farbstoffe | ad 100 | ad 100 | ad 100 | ad 100 | ad 100 |

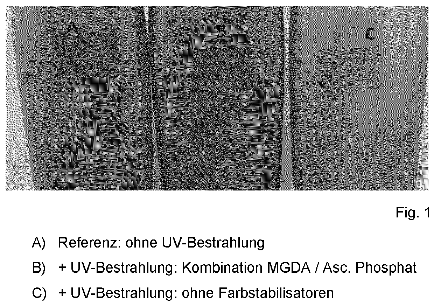

Verschieden gefärbte Kosmetikprodukte wurden unter Laborbedingungen einer extremen Sonnenlichtstrahlung ausgesetzt. Die Proben wurden über 10 Stunden im Sonnenlichtsimulator (Suntest XLS+, Fa. Atlas) bei 750 Watt bestrahlt (dies entspricht ≈150 Stunden direkter Sonnenlicht-Einwirkung). Die erzielten Ergebnisse sind Fig. 1 zu entnehmen (im Original rote Färbung).

## Patentansprüche

1. Stabilisierte Zusammensetzung, enthaltend
a) mindestens einen Komplexbildner der allgemeinen Formel (I) 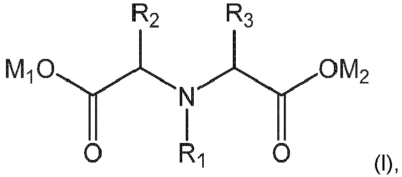 wobei
- R1 für ein Wasserstoffatom, eine lineare oder verzweigte C₁-C₆-Alkylgruppe, eine lineare oder verzweigte Hydroxy-C₂-C₆-alkylgruppe, eine lineare oder verzweigte Carboxy-C₁-C₆-Alkylgruppe oder für ein physiologisch verträgliches Salz hiervon steht,
- R2, R3 unabhängig voneinander für ein Wasserstoffatom, eine lineare oder verzweigte C₁-C₆-Alkylgruppe, eine lineare oder verzweigte Hydroxy-C₂-C₆-alkylgruppe, eine lineare oder verzweigte Carboxy-C₁-C₆-Alkylgruppe oder für ein physiologisch verträgliches Salz hiervon stehen,
- M1, M2 unabhängig voneinander für ein Wasserstoffatom oder ein Äquivalent eines Alkali-, Erdalkali- oder Metallions, bevorzugt für Natrium, Kalium, ½ Magnesium, ½ Calcium, ½ Zink, oder für ein Ammoniumion (NH₄⁺) stehen, und
b) mindestens ein Antioxidans, ausgewählt aus der Gruppe der Ascorbinsäure, Ascorbinsäuresalze, Ascorbylester oder Mischungen davon.

2. Stabilisierte Zusammensetzung nach Anspruch 1, enthaltend Wasser in einem Gewichtsanteil von 5-90 Gew.-% am Gesamtgewicht der Zusammensetzung.

3. Stabilisierte Zusammensetzung nach einem der vorhergehenden Ansprüche, enthaltend
a) einen oder mehrere Komplexbildner gemäß Formel (I) in einem Gewichtsanteil von 0,001-10 Gew.-% am Gesamtgewicht der Zusammensetzung und
b) ein oder mehrere Antioxidantien aus der Gruppe der Gruppe der Ascorbinsäure, Ascorbinsäuresalze, Ascorbylester oder Mischungen davon in einem Gewichtsanteil von 0,0005-1 Gew.-% am Gesamtgewicht der Zusammensetzung.

4. Stabilisierte Zusammensetzung nach einem der vorhergehenden Ansprüche, enthaltend mindestens einen Komplexbildner (a) der allgemeinen Formel (I), wobei
- R1 für eine Gruppe -CH(CH₃)-COOH oder für ein physiologisch verträgliches Salz hiervon steht,
- R2, R3 unabhängig voneinander für eine Carboxymethylgruppe oder für ein physiologisch verträgliches Salz hiervon stehen.

5. Stabilisierte Zusammensetzung nach einem der vorhergehenden Ansprüche, enthaltend mindestens einen Ester der Ascorbinsäure mit einer anorganischen Säure oder einem Salz davon.

6. Stabilisierte Zusammensetzung nach einem der vorhergehenden Ansprüche, enthaltend Sodium Ascorbyl Phosphate, Magnesium Ascorbyl Phosphate oder Mischungen davon.

7. Stabilisierte Zusammensetzung nach einem der vorhergehenden Ansprüche, enthaltend mindestens einen Farbstoff.

8. Stabilisierte kosmetische Zusammensetzung nach einem der vorhergehenden Ansprüche in Form einer Reinigungszubereitung für Haut oder Haare.

9. Verwendung
a) mindestens eines Komplexbildners der allgemeinen Formel (I) 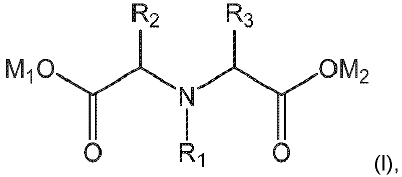 wobei
- R1 für ein Wasserstoffatom, eine lineare oder verzweigte C₁-C₆-Alkylgruppe, eine lineare oder verzweigte Hydroxy-C₂-C₆-alkylgruppe, eine lineare oder verzweigte Carboxy-C₁-C₆-Alkylgruppe oder für ein physiologisch verträgliches Salz hiervon steht,
- R2, R3 unabhängig voneinander für ein Wasserstoffatom, eine lineare oder verzweigte C₁-C₆-Alkylgruppe, eine lineare oder verzweigte Hydroxy-C₂-C₆-alkylgruppe, eine lineare oder verzweigte Carboxy-C₁-C₆-Alkylgruppe oder für ein physiologisch verträgliches Salz hiervon stehen,
- M1, M2 unabhängig voneinander für ein Wasserstoffatom oder ein Äquivalent eines Alkali-, Erdalkali- oder Metallions, bevorzugt für Natrium, Kalium, ½ Magnesium, ½ Calcium, ½ Zink, oder für ein Ammoniumion (NH₄⁺) stehen, und
b) mindestens eines Antioxidans, ausgewählt aus der Gruppe der Ascorbinsäure, Ascorbinsäuresalze, Ascorbylester oder Mischungen davon
zur Stabilisierung von Haushaltsprodukten, kosmetischen oder pharmazeutischen Zusammensetzungen gegen die schädlichen Auswirkungen von Licht, Hitze und/oder Sauerstoff.

10. Verwendung nach Anspruch 9 zur Stabilisierung Farbstoffe enthaltender Haushaltsprodukte, kosmetischer oder pharmazeutischer Zusammensetzungen gegen Farbveränderungen und/oder Entfärbungen.
